# EUROPEAN PATENT APPLICATION

(11) **EP 2 666 516 A1**
(43) Date of publication of application: **27.11.2013**
(21) Application number: 11856316.2
(22) Date of filing: 22.12.2011
(51) Int. Cl.: A61N 5/06, A61B 18/20, A61M 25/01

(54) **LASER THERAPY APPARATUS, LASER THERAPY SYSTEM AND ASSESSMENT METHOD**

(30) Priority: 19.01.2011 JP 2011008733
(71) Applicant: Sony Corporation, Tokyo 108-0075 (JP); Keio University, Tokyo 108-8345 (JP)
(72) Inventor: HAKOMORI, Shiho, Tokyo 108-0075 (JP); ARAI, Tsunenori, Yokohama-shi Kanagawa 223-8522 (JP); ITO, Arisa, Yokohama-shi Kanagawa 223-8522 (JP); TAKAHASHI, Mei, Yokohama-shi Kanagawa 223-8522 (JP)
(74) Representative: Müller - Hoffmann & Partner
(86) International application number: PCT/JP2011/007230
(87) International publication number: WO 2012/098623

(57) **Abstract**

[Object] To provide a laser therapy apparatus that is capable of determining a contact state between a tip portion of a laser catheter and a tissue, which includes a buried state of the tip portion in the tissue, a laser therapy system, and a determination method.

[Solving Means] It includes a connection portion 210 to which a second end portion of a laser catheter 300 is connected, the laser catheter 300 including a first end portion, the second end portion, and an electrode, a laser beam entering and exiting from an apical surface of the first end portion, the electrode being provided at outer periphery of the first end portion along an axial direction of the first end portion, an emission unit 110 configured to emit a laser beam to the second end portion connected to the connection portion 210, an optical state detection unit 130 configured to cause light reflected from the second end portion connected to the connection portion 210 to enter, and to detect an optical state of the entered reflected light, an electrical state detection unit 135 configured to detect an electrical state of the electrode via the second end portion connected to the connection portion 210, and a determination unit 150 configured to determine, based on the detected optical state and electrical state, a contact state between the first end portion and a therapy-target tissue treated by using the laser catheter.

## Description

### Technical Field

The present invention relates to a laser therapy apparatus that treats a therapy-target tissue by using a laser catheter, a laser therapy system, and a determination method that can be applied to the apparatus and the system.

### Background Art

Atrial fibrillation is known as a kind of tachyarrhythmia. A hyperexcited site, which generates an electrical pulse, appears in the vicinity of a junction between a pulmonary vein and a left atrium, and the left atrium minutely vibrates and contracts because of the electrical pulse stimulation, thereby causing atrial fibrillation.

As an atrial fibrillation therapy, the inventors have been proposed application of photodynamic therapy (hereinafter, referred to as "PDT".) (see, for example, Patent Document 1). In PDT, a cardiac muscle tissue, which has absorbed a photosensitive agent, is irradiated with excitation light by using a laser catheter, thereby generating singlet oxygen. The singlet oxygen having strong oxidizing power damages a cardiac muscle tissue, which surrounds the hyperexcited site, thereby forming an electrical conduction block, which blocks conduction of the electrical pulse from the hyperexcited site to the left atrium. As a result, electrical conduction between the hyperexcited site and the left atrium is blocked, and an abnormal vibration and contraction of the left atrium is inhibited.

The photosensitive agent has properties of selectively accumulating in a certain tissue. In view of this, in general, after a predetermined time period (e.g., 8 to 48 hours) passes after a photosensitive agent is administered to a patient, when the state where the concentration of the photosensitive agent is high in a therapy-target tissue and the concentration of the photosensitive agent is low in other tissues and blood is established, i.e., when the state where a so-called photosensitive agent contrast is high is established, irradiation with the excitation light is started. Further, in recent years, PDT in which the accumulating properties of the photosensitive agent are not used and irradiation with the excitation light is started when the photosensitive agent is delivered to the therapy-target tissue by blood is proposed.

In the field of circulatory disease treatment, in order to ensure safety and reliability, it is important to determine a contact state between a tip portion of a laser catheter, which emits excitation light, and a tissue. In Patent Documents 2 and 3, techniques for determining the contact state between the tip portion and the tissue are described. In Patent Document 2, the deformation of the tip portion being in contact with the tissue is estimated by reflected light, thereby detecting the contact state between the tip portion and the tissue. In Patent Document 3, a pressure sensor is provided at the tip portion, thereby detecting the contact state between the tip portion and the tissue.

### Citation List

### Patent Document

Patent Document 1: WO 2008/066126
Patent Document 2: Japanese Unexamined Patent Application Publication No. 2008-531170 (paragraphs [0044] to [0061])
Patent Document 1: Japanese Unexamined Patent Application Publication No. 2009-542371 (paragraph [0085])

### Disclosure of the Invention

### Problem to be solved by the Invention

According to the findings of the inventors, it turns out that such an excessive pressure as to bury the tip portion of the laser catheter in the tissue causes a large burden on a patient during a surgical operation and is deeply involved in a side effect after surgery. However, although it is possible to evaluate the pressure of the tip portion of the laser catheter on the tissue by using the techniques described in Patent Documents 2 and 3, it is difficult for a practitioner such as a doctor to recognize the buried state unless an evaluation standard for a pressure value in the buried state is provided. Furthermore, the relationship between the buried state and the pressure value cannot be uniformly established for all patients, and it is considered that there is an individual difference in the relationship. Moreover, in the techniques described in Patent Documents 2 and 3, because there is a need to mount a device such as a pressure sensor, which is not necessary for treatment, the configuration of the laser catheter is complicated.

In view of the above-mentioned circumstances, it is an object of the present invention to provide a laser therapy apparatus that is capable of determining a contact state between a tip portion of a laser catheter and a tissue, which includes a buried state of the tip portion in the tissue, a laser therapy system, and a determination method.
Another object of the present invention is to provide a laser therapy apparatus that is capable of realizing such determination with a simple configuration, a laser therapy system, and a determination method.

### Means for solving the Problem

In order to achieve the above-mentioned object, a laser therapy apparatus according to an embodiment of the present invention includes a connection portion, an emission unit, an optical state detection unit, an electrical state detection unit, and a determination unit.

To the connection portion, a second end portion of a laser catheter is connected, the laser catheter including a first end portion, the second end portion, and an electrode, a laser beam entering and exiting from an apical surface of the first end portion, the electrode being provided at outer periphery of the first end portion along an axial direction of the first end portion.
The emission unit emits a laser beam to the second end portion connected to the connection portion.
The optical state detection unit causes light reflected from the second end portion connected to the connection portion to enter, and detects an optical state of the entered reflected light.
The electrical state detection unit detects an electrical state of the electrode via the second end portion connected to the connection portion.

The determination unit determines, based on the detected optical state and electrical state, a contact state between the first end portion and a therapy-target tissue treated by using the laser catheter.

Here, in the present invention, the optical state detection unit may detect an intensity of the entered reflected light, the electrical state detection unit may detect a potential of the electrode, and the determination unit may determine that the contact state between the first end portion and the therapy-target tissue is normal in the case where the detected intensity of light is low and the detected potential is low, determine that the first end portion is buried in the therapy-target tissue in the case where the detected intensity of light is low and the detected potential is high, and determine that the laser catheter is in contact with the therapy-target tissue therealong in the case where the detected intensity of light is high and the detected potential is high.

In the present invention, it is possible to determine a contact state between a tip portion (first end portion) of a laser catheter and a tissue, which includes a buried state of the tip portion in the tissue by detecting an optical state of light reflected from the laser catheter and detecting an electrical state of an electrode provided at outer periphery of the first end portion of the laser catheter along an axial direction of the first end portion. Moreover, the electrode is originally used for treatment, and there is no need to mount a device such as a pressure sensor, which is not necessary for treatment. Therefore, it is possible to realize determination of the contact state, which includes the buried state, with a simple configuration.
A laser therapy system according to an embodiment of the present invention includes a laser catheter, a connection portion, an emission unit, an optical state detection unit, an electrical state detection unit, and a determination unit.

The laser catheter includes a first end portion, a laser beam entering and exiting from an apical surface of the first end portion, a second end portion, and an electrode provided at outer periphery of the first end portion along an axial direction of the first end portion.
To the connection portion, the second end portion of the laser catheter is connected.
The emission unit emits a laser beam to the second end portion connected to the connection portion.
The optical state detection unit causes light reflected from the second end portion connected to the connection portion to enter, and detects an optical state of the entered reflected light.
The electrical state detection unit detects an electrical state of the electrode via the second end portion connected to the connection portion.

The determination unit determines, based on the detected optical state and electrical state, a contact state between the first end portion and a therapy-target tissue treated by using the laser catheter.
Here, the electrode provided on the laser catheter may include a ring electrode.

A determination method according to an embodiment of the present invention includes emitting a laser beam to a second end portion of a laser catheter, the laser catheter including a first end portion, the second end portion, and an electrode, a laser beam entering and exiting from an apical surface of the first end portion, the electrode being provided at outer periphery of the first end portion along an axial direction of the first end portion, causing light reflected from the second end portion to enter, and detecting an optical state of the entered reflected light, detecting an electrical state of the electrode, and determining, based on the detected optical state and electrical state, a contact state between the first end portion and a therapy-target tissue treated by using the laser catheter.

### Effect of the Invention

In the present invention, it is possible to determine a contact state between a tip portion of a laser catheter and a tissue, which includes a buried state of the tip portion in the tissue. Moreover, it is possible to realize determination of the contact state, which includes the buried state, with a simple configuration.

### Brief Description of Drawings

[Fig. 1] A schematic diagram showing the entire system including a PDT apparatus according to an embodiment of the present invention.
[Fig. 2] A schematic diagram showing a laser catheter inserted in a heart.
[Fig. 3] A block diagram showing a configuration of the PDT apparatus.
[Fig. 4] A diagram showing a diagram showing the appearance of the laser catheter.
[Fig. 5] A partial cross-sectional view of a tip portion of the laser catheter shown in Fig. 4.
[Fig. 6] A cross-sectional view taken along the line A-A in Fig. 5.
[Fig. 7] A schematic diagram showing a contact state between the tip portion of the laser catheter and a tissue.
[Fig. 8] A table showing a relationship between a fluorescence intensity and potential and the contact state. Mode(s) for Carrying Out the Invention

Hereinafter, embodiments according to the present invention will be described with reference to the drawings. In the embodiments, a description will be given using, as a laser apparatus, a photodynamic therapy apparatus (hereinafter referred to as "PDT apparatus").

### [Configuration of Entire System]

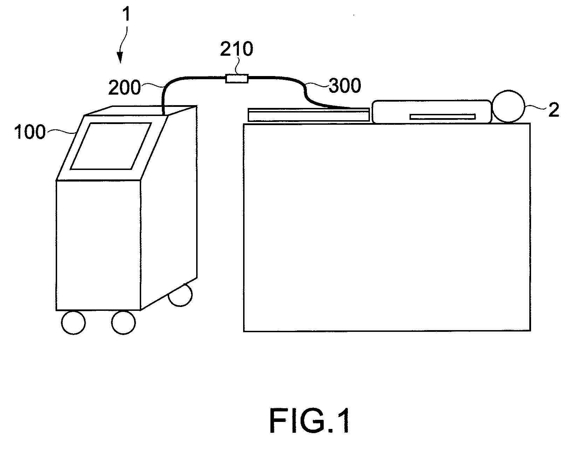

Fig. 1 is a schematic diagram showing the entire therapy system including a PDT apparatus according to an embodiment of the present invention.

A PDT apparatus 1 includes a PDT apparatus main body 100, a tube 200 connected to the PDT apparatus main body 100, and a connector (connection portion) 210 provided at the tip of the tube 200.

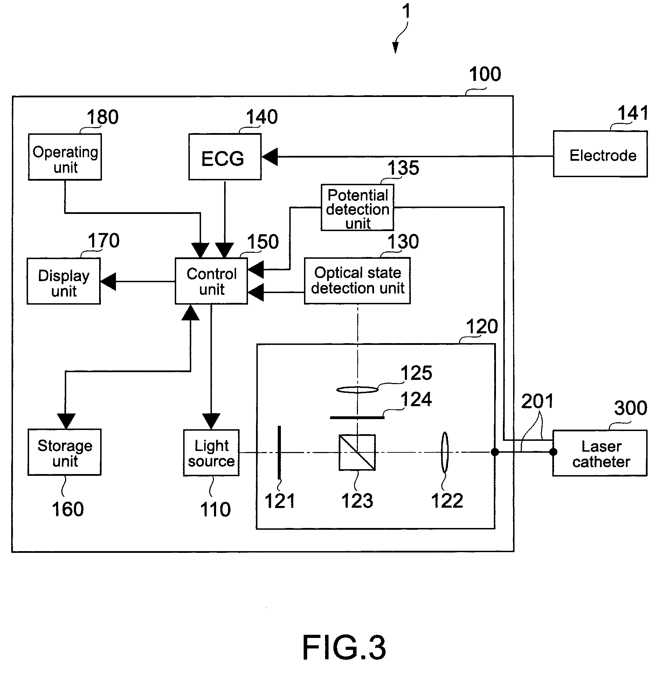

The tube 200 is a soft hollow tube, and is capable of transmitting light through a built-in apparatus-attached optical fiber 201 (see Fig. 3). Moreover, through a built-in apparatus-attached electrical wiring 202 (see Fig. 3), it is possible to, for example, measure a potential in the tip portion of a laser catheter 300.
To the connector 210, the laser catheter 300 is detachably connected.

To a patient 2, a photosensitive agent is administered. In the case of being administered by intravenous injection, the administered photosensitive agent diffuses in the blood, and then diffuses in a tissue such as a cardiac muscle tissue. A dose of photosensitive agent necessary for treatment may be administered at one time by intravenous injection, may be continuously administered by intravenous drip, may be administered at one time or continuously via the oral route, or may be locally administered. The photosensitive agent is an agent that is excited by absorbing light having a certain wavelength, and emits fluorescence. Examples of the photosensitive agent include an agent called talaporfin sodium (Laserphyrin (registered trademark), Meiji Seika Kaisha Ltd.). Because the Q-band absorption wavelength of the agent is near 664 nm, an excitation light source for the agent with, for example, 600 to 800 nm, favorably 660 to 680 nm, or more favorably 664 plus or minus 2 nm is used.

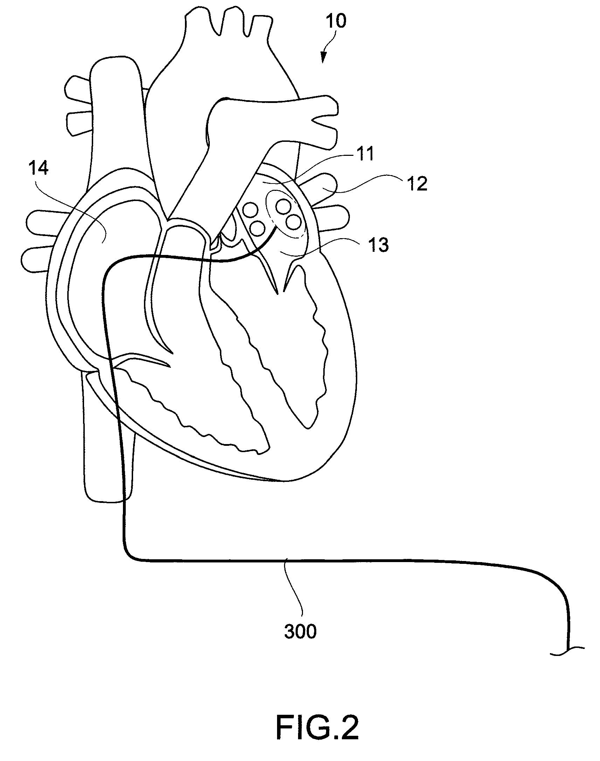

Fig. 2 is a schematic diagram showing a laser catheter inserted in a heart.

The laser catheter 300 is inserted in a right atrium 14 of a heart 10 through a femoral vein or a jugular vein of the patient 2. The laser catheter 300, which has reached the right atrium 14, penetrates a septum, and is led to a left atrium 13.

### [Configuration of PDT Apparatus Main Body]

Fig. 3 is a block diagram showing the PDT apparatus main body.

The PDT apparatus main body 100 includes a light source 110, an optical system 120, an optical state detection unit 130, a potential detection unit 135, an electrocardiograph 140, a control unit 150, a storage unit 160, a display unit 170, and an operating unit 180.

The light source 110 outputs excitation light of a photosensitive agent. The wavelength of the light output by the light source 110 is the same as the Q-band absorption wavelength of the photosensitive agent. For example, in the case where a photosensitive agent whose Q-band absorption wavelength is near 664 nm is used, a semiconductor laser with the oscillation wavelength of 600 to 800 nm, favorably 660 to 680 nm, or more favorably 664 plus or minus 2 nm is used as the light source 110. The excitation light (laser light) output by the light source 110 enters the laser catheter 300 via the optical system 120.

The optical system 120 causes the excitation light, which is emitted from the light source 110, to enter the laser catheter 300, which is connected to the connector 210 through the apparatus-attached optical fiber 201. The optical system 120 extracts, from the laser catheter 300, fluorescence (reflected light) emitted from a photosensitive agent, which is irradiated with the excitation light, and causes the fluorescence to enter the optical state detection unit 130.
The optical system 120 includes a short pass filter 121, a first lens 122, a polarizing beam splitter (hereinafter referred to as "PBS".) 123, a long pass filter 124, and a second lens 125.

The short pass filter 121 is a short-wavelength transmission filter with a cut-on wavelength of 670 nm, and cuts long-wavelength radiation of a laser beam. The excitation light from the light source 110 has a radiation component in the fluorescence observation wavelength range (long-wavelength side of peak wavelength). In view of the above, the radiation component of the excitation light on the long-wavelength side is cut at the stage prior to collecting the light in the laser catheter 300. The excitation light, which has been transmitted through the short pass filter 121, enters the first lens 122.

The first lens 122 collects the excitation light, which has entered from the short pass filter 121, on an end surface of the laser catheter 300. Further, the first lens 122 collects fluorescence from the tip portion of the laser catheter 300 on the PBS 123. It should be noted that a part of the excitation light from the light source 110 is reflected on an end surface of the apparatus-attached optical fiber 201 at the PDT apparatus main body 100 side, on the inside of the connector 210, and on the tip portion of the laser catheter 300, and enters the PBS 123 as specular reflection light. The specular reflection light is noisy when detecting fluorescence.

By using polarization differences, the PBS 123 causes the specular reflection light, which has reflected on an end surface of the optical fiber in the tube 200, out of the light having entered from the first lens 122, to transmit therethrough, does not detect the specular reflection light, reflects the fluorescence and the specular reflection light reflected on the other end surfaces, and leads them to a detector. The fluorescence, which has been transmitted through the PBS 123, enters the long pass filter 124.

The long pass filter 124 causes the specular reflection light, which has reflected on the inside of the connector 210 and the tip portion of the laser catheter 300, out of the light having entered from the PBS 123, not to transmit therethrough, causes only the fluorescence to transmit therethrough, and leads the fluorescence to the detector. The fluorescence, which has been transmitted through the long pass filter 124, enters the second lens 125.
The second lens 125 collects the fluorescence, which has entered from the long pass filter 124, on the optical state detection unit 130.

The optical state detection unit 130 is, for example, a linear image sensor, and spectroscopically detects the fluorescence having entered from the optical system 120. That is, the optical state detection unit 130 detects the light having the excitation wavelength, and detects the fluorescence of the photosensitive agent, which is light having a wavelength longer than the excitation wavelength. The optical state detection unit 130 outputs, as an electrical signal, an intensity of the detected fluorescence to the control unit 150.

The potential detection unit 135 detects the potential of a second electrode (to be described later) with respect to a first electrode on the tip portion of the laser catheter 300. The potential detection unit 135 output, as an electrical signal, the detected potential to the control unit 150.

To the electrocardiograph 140, an electrode pad 141 is connected via an electrode code (not shown). The electrocardiograph 140 obtains an electrocardiographic signal of the patient 2 via the electrode pad 141, which is attached to the patient 2, and via the electrode code, and supplies the obtained electrocardiographic signal to the control unit 150.
The control unit 150 controls the respective units of the PDT apparatus 1.

The control unit 150 determines a contact state between the laser catheter 300 and a tissue, which includes a buried state of the laser catheter 300 in the tissue, based on the electrical signal obtained from the optical state detection unit 130 and the potential detection unit 135.

The control unit 150 determines whether or not an electrical conduction block is formed based on the electrical signal obtained from the optical state detection unit 130 and the electrocardiographic signal obtained from the electrocardiograph 140.
The control unit150 outputs a display instruction to display information of the determination result of the contact state including the buried state.
The storage unit 160 is a non-volatile memory, and is set in, for example, a flash memory, an HDD (Hard Disk Drive), or another solid memory.

The display unit 170 is a display device, which uses, for example, a liquid-crystal display device. When obtaining a display instruction from the control unit 150, the display unit 170 displays, on a display screen, for example, information of the determination result of the contact state including the buried state, based on display information included in the display instruction.

The operating unit 180 receives an instruction by an input operation from a practitioner, and outputs the received instruction to the control unit 150. Examples of the instruction include an instruction to turn on/off the excitation light output from the light source 110, and an instruction to change the intensity. As the intensity of the excitation light, it is possible to select at least one of two levels of intensity including a first intensity, which has a low power (e.g., optical output of 1 mW or less) and is minimally-invasive with respect to a tissue and blood, and a second intensity, which has a high power and is approximately 1,000 times higher than the first intensity. The first intensity is selected when monitoring the agent concentration and the contact state of the laser catheter 300 before treatment. The second intensity is selected when treatment is actually performed.

### [Configuration of Laser Catheter]

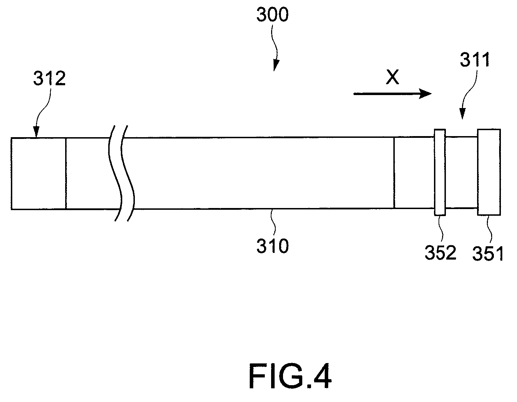

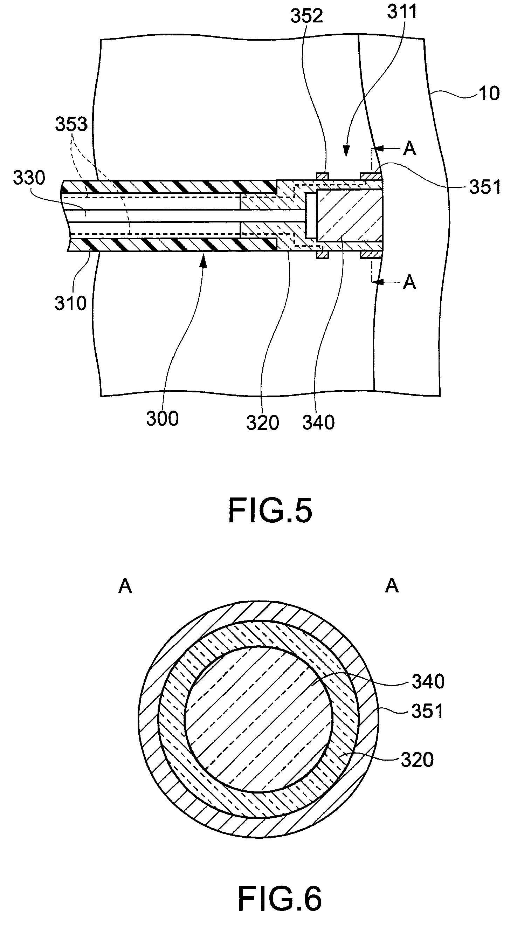

Fig. 4 is a diagram showing the appearance of the laser catheter, Fig. 5 is a partial cross-sectional view of a tip portion of the laser catheter shown in Fig. 4, and Fig. 6 is a cross-sectional view taken along the line A-A in Fig. 5.

The laser catheter 300 emits excitation light from an end surface of the tip portion (first end portion). The laser catheter 300 includes a catheter tube 310, a first end portion 311, a second end portion 312, a holding portion 320, an optical fiber 330, an optical window 340, a plurality of electrodes 351 and 352, and a wiring 353.

The catheter tube 310 is a soft hollow tube, and is led to the inner wall of a cardiac muscle tissue of the heart 10 of the patient 2. The catheter tube 310 has the optical fiber 330 and the wiring 353 therein. The catheter tube 310 includes the first end portion 311 and the second end portion 312 at both ends thereof.

The holding portion 320 is provided on the first end portion 311 of the laser catheter 300. The holding portion 320 holds the optical fiber 330 and the optical window 340 with respect to the catheter tube 310. At the outer periphery of the holding portion 320 being the first end portion 311 along the axial direction (X direction in Fig. 4), the plurality of electrodes 351 and 352 are provided so as to have a predetermined space t between them. The plurality of electrodes 351 and 352 include Pt (platinum) and have a ring shape, for example. Out of the plurality of electrodes 351 and 352, the electrode 351 is located at the edge portion of the holding portion 320, for example. The electrode 351 may cover an end surface of the holding portion 320. Conversely, the electrode 351 may be located so as to have a small space between the electrode 351 and the edge portion of the holding portion 320. For example, out of the plurality of electrodes 351 and 352, the electrode 351 has a width wider than the electrode 352, and the space t between the plurality of electrodes 351 and 352 is approximately 2 mm. The space t between the plurality of electrodes 351 and 352 and other dimensions may be variable as long as the change in potential of the second electrode with respect to the first electrode when the plurality of electrodes 351 and 352 are brought into contact with a tissue can be detected. The plurality of electrodes 351 and 352 are brought into contact with a tissue when the first end portion 311 of the laser catheter 300 is buried in the tissue.

The plurality of electrodes 351 and 352 are connected to the apparatus-attached electrical wiring 202 at the second end portion 312 via the respective wirings 353, and connected to the potential detection unit 135 of the PDT apparatus main body 100. For example, the potential detection unit 135 detects potential information of a cardiac muscle tissue from an electrode, which is brought into contact with the cardiac muscle tissue. Further, the potential detection unit 135 may detect the potential difference between the pair of the electrodes 351 and 352 by applying predetermined voltage to the electrode 351, and detecting the potential of the electrode 352. In the case where the electrode 352 is not in contact with the tissue, the detected potential of the electrode 352 is low, and in the case where the electrode 352 is not in contact with the tissue, the detected potential of the electrode 352 is high.

The optical fiber 330 is, for example, one quartz step index fiber having a core diameter of 133 µm and an outside diameter of 500 µm or having a core diameter of 200 µm and an outside diameter of 350 µm. The optical fiber 330 transmits the excitation light from the PDT apparatus 1. The optical fiber 330 emits the transmitted excitation light, as an irradiation light 301, from the tip to the optical window 340. The beam diameter of the irradiation light 301 increases with the angle determined by the numerical aperture (NA) of the optical fiber 330. The tip of the optical fiber 330 is processed so that the beam diameter of the irradiation light 301 appropriately increases. The optical fiber 330 transmits the fluorescence, which is emitted from a photosensitive agent absorbed in a tissue and irradiated with excitation light, to the PDT apparatus 1.

The optical window 340 is provided on the outermost of the tip portion of the laser catheter 300 so that the optical window 340 is optically connected to the tip of the optical fiber 330. The optical window 340 includes a solid transparent material, e.g., a glass material such as BK7. The optical window 340 causes the irradiation light 301, which is output from the tip of the optical fiber 330, to transmit therethrough. The optical window 340 collects the fluorescence, which is emitted from the photosensitive agent, on the tip of the optical fiber 330.

### [Operation of PDT Apparatus]

Next, an operation of the PDT apparatus 1 configured as described above will be described.

The operation of the PDT apparatus 1 will be described in the following order.
(1) Preparation for PDT
(2) Contact-Monitoring Operation

In the contact-monitoring operation, the light source 110 outputs excitation light with the first intensity, and the control unit 150 determines a contact state between the laser catheter 300 and an inner wall of a tissue, which includes a buried state of the laser catheter 300 in the inner wall of the tissue based on the fluorescence intensity detected by the optical state detection unit 130 and the potential detected by the potential detection unit 135.

### (3) Laser Therapy Performing Operation

In the laser therapy performing operation, the light source 110 outputs the excitation light with the second intensity, and laser therapy is actually performed. Also in the operation, as in the contact-monitoring operation, the contact state between the laser catheter 300 and the inner wall of the tissue, which includes the buried state of the laser catheter 300 in the inner wall of the tissue, is determined.
In the following, the operations are described in more detail.

### [(1) Preparation for PDT]

First, a practitioner such as a doctor inserts the laser catheter 300 in the heart 10 through a femoral vein or a jugular vein of the patient 2. The tip portion of the laser catheter 300 is disposed in the vicinity of a pulmonary vein 12 of an inner wall of a cardiac muscle tissue 11 of the left atrium 13 (see Fig. 2).
Subsequently, with reference to various types of reference data, the practitioner administers a photosensitive agent to the patient 2.

### [(2) Contact-Monitoring Operation]

Subsequently, the contact-monitoring operation is performed.

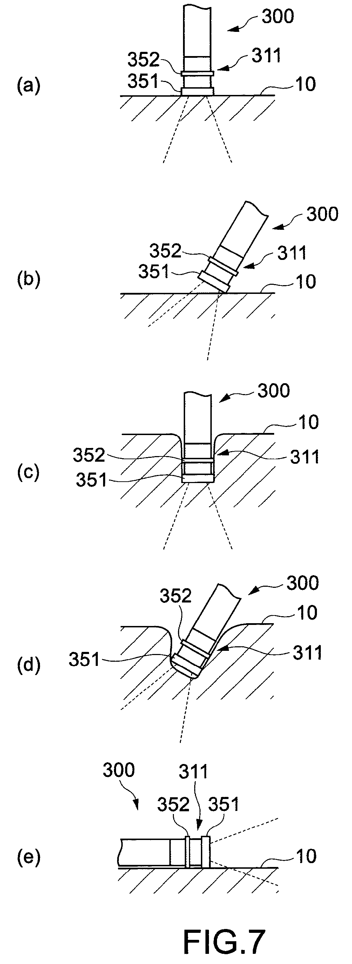

Fig. 7 is a schematic diagram showing a contact state of a laser catheter.

The laser catheter 300 is desirably disposed such that the tip portion (first end portion) as a light-emitting portion is vertically brought into contact with the inner wall of the cardiac muscle tissue 11 (see Fig. 7(a), hereinafter referred to as "vertical contact state".). This is because an intra-atrial blood 15 is removed from the tip portion of the laser catheter 300, and activation of a photosensitive agent in the intra-atrial blood 15 is suppressed. Further, this is because the photo-sensitive agent absorbed in a tissue is selectively activated by directly bringing the tip portion of the laser catheter 300 into contact with the tissue.

However, actually, the tip portion of the laser catheter 300 is not vertically but obliquely, as shown in Fig. 7(b), brought into contact with the inner wall of the cardiac muscle tissue 11 in some cases. As shown in Fig. 7(c), the tip portion of the laser catheter 300 is buried in the inner wall of the cardiac muscle tissue 11 in some cases. Moreover, as shown in Fig. 7(d), the tip portion of the laser catheter 300 is obliquely buried in the inner wall of the cardiac muscle tissue 11 in some cases. As shown in Fig. 7(e), the tip portion of the laser catheter 300 is not even brought into contact with the inner wall of the cardiac muscle tissue 11 in some cases.

In particular, as shown in Fig. 7(c) and 7(d), in the case where the tip portion of the laser catheter 300 is buried in the inner wall of the cardiac muscle tissue 11, the tip portion of the laser catheter 300 is excessively pressed onto the inner wall of the cardiac muscle tissue 11. Such an excessive pressure causes a large burden on a patient during a surgical operation and is deeply involved in a side effect after surgery. In view of the above, in the contact-monitoring operation according to this embodiment, the control unit 150 determines a contact state between the laser catheter 300 and an inner wall of a tissue, which includes a buried state of the laser catheter 300 in the inner wall of the tissue based on the fluorescence intensity detected by the optical state detection unit 130 and the potential detected by the potential detection unit 135.

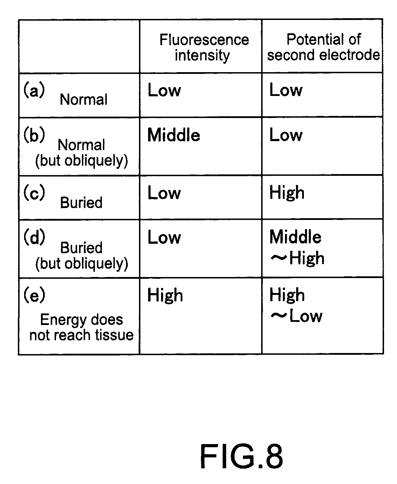

Fig. 8 is a table showing a relationship between a detected fluorescent intensity and potential and the contact state shown in Fig. 7(a) to (e).

As shown in Fig. 7(a), in the case where the tip portion of the laser catheter 300 is vertically brought into contact with the inner wall of the cardiac muscle tissue 11, blood (having agent concentration higher than the tissue) between the tip portion and the inner wall of the cardiac muscle tissue 11 is almost removed. Therefore, the fluorescence intensity detected by the optical state detection unit 130 is low. In this case, because the electrode 352 is not in contact with the tissue, the potential of the electrode 352 detected by the potential detection unit 135 is low.

As shown in Fig. 7(b), in the case where the tip portion of the laser catheter 300 is obliquely brought into contact with the inner wall of the cardiac muscle tissue 11, a small amount of blood lies between the tip portion and the inner wall of the cardiac muscle tissue 11. Therefore, the fluorescence intensity detected by the optical state detection unit 130 is middle. In this case, because the electrode 352 is not in contact with the tissue, the potential of the electrode 352 detected by the potential detection unit 135 is low.

As shown in Fig. 7(c), in the case where the tip portion of the laser catheter 300 is vertically buried in the inner wall of the cardiac muscle tissue 11, blood between the tip portion and the inner wall of the cardiac muscle tissue 11 is almost removed. Therefore, the fluorescence intensity detected by the optical state detection unit 130 is low. In this case, because the electrode 352 is in contact with the tissue, the potential of the electrode 352 detected by the potential detection unit 135 is high.

As shown in Fig. 7(d), in the case where the tip portion of the laser catheter 300 is obliquely buried in the inner wall of the cardiac muscle tissue 11, blood between the tip portion and the inner wall of the cardiac muscle tissue 11 is almost removed. Therefore, the fluorescence intensity detected by the optical state detection unit 130 is low. In this case, because the electrode 352 is slightly in contact with the tissue, the potential of the electrode 352 detected by the potential detection unit 135 is middle to high.

In any case, in the case where the tip portion of the laser catheter 300 is buried in the inner wall of the cardiac muscle tissue 11, the potential of the electrode 352 detected by the potential detection unit 135 is not low. Therefore, it is possible to determine the buried state by detecting that the potential of the electrode 352 is not low by the potential detection unit 135.

As shown in Fig. 7(e), in the case where the tip portion of the laser catheter 300 is not in contact with the inner wall of the cardiac muscle tissue 11, blood lies at the tip portion in almost all cases. Therefore, the fluorescence intensity detected by the optical state detection unit 130 is high. In this case, in the case where the electrode 352 is in contact with the tissue, the potential of the electrode 352 detected by the potential detection unit 135 is high, and in the case where the electrode 352 is slightly in contact with the tissue, the potential of the electrode 352 detected by the potential detection unit 135 is low. In any case, in the case where the tip portion of the laser catheter 300 is not in contact with the inner wall of the cardiac muscle tissue 11, the fluorescence intensity detected by the optical state detection unit 130 is high. Therefore, it is possible to determine the contact state of the catheter, which is in contact with the cardiac muscle tissue therealong, by detecting that the fluorescence intensity is high by the optical state detection unit 130. Moreover, by using only the information from the potential detection unit 135, it is difficult to understand the difference between the state of (e) (state where the electrode 352 is in contact with the cardiac muscle tissue) and the states of (c) and (d). However, by combining information of the fluorescence intensity, it is possible to determine the state of the tip portion.

Based on the electrical signal obtained from the optical state detection unit 130 and the potential detection unit 135, the control unit 150 determines the contact state between the laser catheter 300 and the tissue, which includes the buried state of the laser catheter 300 in the tissue, i.e., which state shown in Fig. 7(a) to (e) the laser catheter 300 is in. For example, in the case where the laser catheter 300 is in any one of the states shown in Fig. 7(c) to (e), the control unit 150 displays a predetermined warning on the display unit 170. At this time, the control unit 150 may display the warning so as to distinguish Fig. 7(c) and (d), and Fig. 7 (e). Accordingly, a practitioner such as a doctor can recognize that the laser catheter 300 is in the buried state, and an excessive pressure on the cardiac muscle tissue can be alleviated. Therefore, a side effect can be reduced, which leads to reduction of a burden on a patient. Further, it is possible to prevent an effect on the surrounding organs. It should be noted that the practitioner such as a doctor may be notified of the warning by not display on the display unit but by a predetermined alarm sound. The practitioner operates a handpiece or the like (not shown) provided on the laser catheter 300, thereby changing the contact state between the tip portion of the laser catheter 300 and the tissue.

### [(3) Laser Therapy Performing Operation]

The practitioner operates the operating unit 180, thereby inputting an excitation-light-output instruction with the high-power second intensity to the control unit 150. When obtaining the excitation-light-output instruction, the control unit 150 outputs the excitation-light-output instruction with the second intensity to the light source 110. When obtaining the excitation-light-output instruction from the control unit 150, the light source 110 outputs the excitation light with the second intensity. A tissue is irradiated with the excitation light output from the light source 110 via the optical system 120 and the laser catheter 300. Thus, photodynamic therapy is performed.

Also in the operation, as in the contact-monitoring operation, a contact state between the laser catheter 300 and an inner wall of a tissue, which includes a buried state of the laser catheter 300 in the inner wall of the tissue, is determined. That is, the control unit 150 determines the contact state between the laser catheter 300 and the tissue, which includes the buried state of the laser catheter 300 in the tissue, i.e., which state shown in Fig. 7(a) to (e) the laser catheter 300 is in, based on the electrical signal obtained from the optical state detection unit 130 and the potential detection unit 135. The control unit 150 displays a predetermined warning on the display unit 170. Accordingly, similarly to the above, a practitioner such as a doctor can recognize that the laser catheter 300 is in the buried state, and an excessive pressure on the cardiac muscle tissue can be alleviated.

In this embodiment, the plurality of electrodes 351 and 352 are originally used for treatment (e.g., simple treatment effect and determination of the contact state), and there is no need to mount a device such as a pressure sensor, which is not necessary for treatment. Therefore, it is possible to realize determination of the contact state, which includes the buried state, with a simple configuration.
Embodiments according to the present invention are not limited to the above-mentioned embodiments and various modifications can be made.

In the above-mentioned embodiment, the laser catheter 300 is detachably connected to the connector 210 of the PDT apparatus 1. However, the laser catheter 300 may be integrally provided on the PDT apparatus 1.

In the above-mentioned embodiment, the tube 200 is provided on the PDT apparatus main body 100, and the connector 210 is provided at the tip of the tube 200. However, the connector 210 may be provided on the PDT apparatus main body 100.
In the above-mentioned embodiment, a fluorescence intensity has been described as an exemplary optical state to be detected. However, as the optical state, for example, a difference in a diffuse reflection light intensity (of excitation light) of a tissue and blood may be used.
In the above-mentioned embodiment, a potential has been described as an exemplary electrical state to be detected. However, as the electrical state, for example, resistance may be used.
In the above-mentioned embodiment, the two electrodes 351 and 352 have been described as an example, one electrode or three or more electrodes may be used.
In the above-mentioned embodiment, an electrode having a ring shape has been described as an example. However, the electrode may have a pectinate shape or a sawtooth shape. Description of Reference Numerals

- 1: photodynamic therapy (PDT) apparatus
- 100: PDT apparatus main body
- 110: light source (emission unit)
- 130: optical state detection unit
- 135: potential detection unit (electrical state detection unit)
- 150: control unit (determination unit)
- 210: connector (connection portion)
- 300: laser catheter
- 311: first end portion
- 312: second end portion
- 351, 352: electrode

## Claims

1. A laser therapy apparatus, comprising:
a connection portion to which a second end portion of a laser catheter is connected, the laser catheter including a first end portion, the second end portion, and an electrode, a laser beam entering and exiting from an apical surface of the first end portion, the electrode being provided at outer periphery of the first end portion along an axial direction of the first end portion;
an emission unit configured to emit a laser beam to the second end portion connected to the connection portion;
an optical state detection unit configured
to cause light reflected from the second end portion connected to the connection portion to enter, and
to detect an optical state of the entered reflected light;
an electrical state detection unit configured to detect an electrical state of the electrode via the second end portion connected to the connection portion; and
a determination unit configured to determine, based on the detected optical state and electrical state, a contact state between the first end portion and a therapy-target tissue treated by using the laser catheter.

2. The laser therapy apparatus according to claim 1, wherein
the optical state detection unit is configured to detect an intensity of the entered reflected light,
the electrical state detection unit is configured to detect a potential of the electrode, and
the determination unit is configured
to determine that the contact state between the first end portion and the therapy-target tissue is normal in the case where the detected intensity of light is low and the detected potential is low,
to determine that the first end portion is buried in the therapy-target tissue in the case where the detected intensity of light is low and the detected potential is high, and
to determine that the laser catheter is in contact with the therapy-target tissue therealong in the case where the detected intensity of light is high and the detected potential is high.

3. A laser therapy system, comprising:
a laser catheter including
a first end portion, a laser beam entering and exiting from an apical surface of the first end portion
a second end portion, and
an electrode provided at outer periphery of the first end portion along an axial direction of the first end portion;
a connection portion to which the second end portion of the laser catheter is connected;
an emission unit configured to emit a laser beam to the second end portion connected to the connection portion;
an optical state detection unit configured
to cause light reflected from the second end portion connected to the connection portion to enter, and
to detect an optical state of the entered reflected light;
an electrical state detection unit configured to detect an electrical state of the electrode via the second end portion connected to the connection portion; and
a determination unit configured to determine, based on the detected optical state and electrical state, a contact state between the first end portion and a therapy-target tissue treated by using the laser catheter.

4. The laser therapy system according to claim 3, wherein
the electrode provided on the laser catheter is a ring electrode.

5. A determination method, comprising:
emitting a laser beam to a second end portion of a laser catheter, the laser catheter including a first end portion, the second end portion, and an electrode, a laser beam entering and exiting from an apical surface of the first end portion, the electrode being provided at outer periphery of the first end portion along an axial direction of the first end portion;
causing light reflected from the second end portion to enter, and detecting an optical state of the entered reflected light;
detecting an electrical state of the electrode; and
determining, based on the detected optical state and electrical state, a contact state between the first end portion and a therapy-target tissue treated by using the laser catheter.
